(19) Europäisches Patentamt
European Patent Office
Office européen des brevets

(11) **EP 4 217 059 B1**

(12) **EUROPEAN PATENT SPECIFICATION**

(45) Date of publication and mention
of the grant of the patent:
**19.11.2025 Bulletin 2025/47**

(21) Application number: **21778486.7**

(22) Date of filing: **21.09.2021**

(51) International Patent Classification (IPC):
**A61P 3/10** *(2006.01)*    **A23L 29/281** *(2016.01)*
**A23L 33/00** *(2016.01)*    **A61K 38/39** *(2006.01)*
**C07K 14/78** *(2006.01)*

(52) Cooperative Patent Classification (CPC):
**A61P 3/10; A23L 33/28; A23L 33/30; A61K 38/39;
C07K 14/78**

(86) International application number:
**PCT/EP2021/075918**

(87) International publication number:
**WO 2022/063766 (31.03.2022 Gazette 2022/13)**

(54) **DRY COLLAGEN POWDER WITH SATIATING PROPERTIES AND METHOD FOR ITS PREPARATION**

TROCKENES KOLLAGENPULVER MIT SÄTTIGUNGSEIGENSCHAFTEN UND VERFAHREN ZU SEINER HERSTELLUNG

POUDRE DE COLLAGÈNE SEC AUX PROPRIÉTÉS RASSASIANTES ET SON PROCÉDÉ DE PRÉPARATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.09.2020 ES 202030966**

(43) Date of publication of application:
**02.08.2023 Bulletin 2023/31**

(73) Proprietor: **Viscofan, S.A.
31192 Tajonar (Navarra) (ES)**

(72) Inventors:
• **GONZÁLEZ SALAZAR, Itxaso**
**31192 TAJONAR (Navarra) (ES)**
• **SORET MARTÓN, Agustín**
**31192 TAJONAR (Navarra) (ES)**
• **LONGO ARESO, Carlos, María**
**31192 TAJONAR (Navarra) (ES)**
• **RECALDE IRURZUN, José, Ignacio**
**31192 TAJONAR (Navarra) (ES)**
• **IZCO ZARATIEGUI, Jesús, María**
**31192 TAJONAR (Navarra) (ES)**

(74) Representative: **Herrero & Asociados, S.L.
Edificio Aqua - Calle Agustín de Foxá, 4-10, 2OA
28036 Madrid (ES)**

(56) References cited:
EP-A1- 1 270 672       WO-A1-2018/209008
CN-A- 104 628 844      CN-A- 106 367 460
CN-A- 107 912 543      US-A- 3 932 677
US-A- 5 153 067        US-A1- 2011 039 767
US-A1- 2011 135 699    US-A1- 2013 210 713
US-A1- 2013 303 448    US-A1- 2014 303 347
US-B2- 9 050 296

• WOO MINJI ET AL: "Anti-Obesity Effects of
Collagen Peptide Derived from Skate (Raja
kenojei) Skin Through Regulation of Lipid
Metabolism", MARINE DRUGS, vol. 16, no. 9, 30
August 2018 (2018-08-30), Basel, CH, pages 306,
XP055964443, ISSN: 1660-3397, DOI: 10.3390/
md16090306

**Description**

**FIELD OF THE INVENTION**

The present invention belongs to the dietary field of satiating products. More particularly, the invention relates to a dry collagen powder with satiating activity that can be used as a food additive or incorporated into a food product to increase its satiating power. The invention is also directed to the method of preparing satiating powder, as well as its application in dietary and/or medical treatments in humans or animals to increase satiety and reduce the feeling of hunger and, in this way, reduce food intake. The dry collagen powder of the invention is therefore useful for combating obesity.

The invention is also related to any use as a satiating agent or as a food supplement or as an ingredient in substitute products, food for medical use or novel food that comprises said collagen powder for weight reduction, as well as with a food product that comprises the aforementioned collagen powder.

**BACKGROUND OF THE INVENTION**

Obesity and its associated disorders are a public health problem worldwide. In this sense, different strategies have been developed to combat it, among which increasing metabolism, inhibiting digestion and/or reducing calorie intake are included.

The use of satiating products or additives whose purpose is to reduce appetite and thus minimize food intake is within the strategy of reducing calorie intake. The satiating effect occurs naturally with food intake and lasts as long as the stomach is not emptied. Therefore, products that persist longer in the stomach and are digested more slowly will have a longer satiating effect. Also the "swelling" or swelling effect favors the satiating effect by increasing the pressure on the walls of the digestive system.

Products with a satiating effect have been developed based on different compounds or substances and also based on different principles.

One approach to achieving the satiating effect is by increasing the pressure on the stomach walls. Thus, DE10161986 describes a composition for oral administration based on a solid, liquid or gas that under the conditions of the stomach releases gas by increasing the pressure on the stomach walls and producing the satiating effect. This satiating effect, however, is short-lived and therefore ineffective.

Another strategy has been based on the use of polymers and/or polysaccharides with swelling capacity to provide the satiating effect.

For example, WO2009049105 describes a method to induce satiety through the use of polymers that increase the volume of the cud without increasing the energy density thereof.

For its part, WO2004056375 refers to an agent to produce a satiating effect and to reduce the level of cholesterol that contains poorly esterified polysaccharides and at least one polysaccharide capable of swelling and increasing its volume.

Similarly, WO2004056393 describes a satiating agent based on polysaccharides containing polyuronic acid capable of swelling in the stomach.

Other strategies are aimed at making the components of the satiating products more resistant to acid hydrolysis in the stomach so that they exert their satiating effect on the duodenum. For example, EP 2651426 describes a product based on pea and/or wheat protein incorporated in a gastro-resistant vehicle so that said proteins reach the duodenum without hydrolyzing and exert their satiating function there.

Collagen or its derivatives have also been used to develop products or compositions with a satiating effect and as a means to combat obesity.

Thus, KR100417020 describes a composition with a satiating effect based on agar, alginate gel and gelatin from collagen hydrolysis.

EP0901792 describes a compressed and non-toxic vehicle that once in the stomach expands and has a sponge-like shape, thus producing the satiating effect. This vehicle comes from marine organisms like sponges and has a partially collagen structure.

Document CN106509126 describes a drink for weight loss and slimming in the form of a water-reconstitutable powder comprising a complex mixture of multiple components and nutrients. One of these components is collagen powder.

EP2575496 describes a collagen powder with satiating properties. The collagen powder described in this document is characterized by being partially denatured, losing its native state and containing a high percentage of gelatin.

Document CN107912543A discloses a method for obtaining a powder containing a high amount of native collagen.

The authors of the present invention have developed a collagen powder with satiating properties that can be used as a food or dietary additive, the main characteristic of which is that it has a greater swelling capacity than existing commercial products under pH conditions similar to those of the stomach in digestion, as well as a greater resistance to enzymatic digestion, which allows to extend the satiating effect for longer periods.

The collagen powder of the present invention has not been subjected to any type of denaturation process, therefore, it has a zero or extremely low gelatin level. In fact, the collagen powder of the present invention has a structure very close to that of native collagen.

## OBJECT OF THE INVENTION

The main object of the invention is a dry collagen powder with satiating properties according to claim

From now on we will refer interchangeably to this dry collagen powder as a powder or product of the invention.

Another object of the present invention is a food or dietary ingredient or supplement comprising the powder of the invention. From now on we will refer interchangeably to this ingredient or food or dietary supplement as an ingredient or supplement of the invention. Furthermore, a food product that incorporates, contains or comprises in its composition the powder of the invention or the ingredient or supplement of the invention is also an object of the invention.

Another object of the present invention is a method for obtaining the powder of the invention. We will refer to this as the method of the invention.

A final object of the invention is represented by the use of the powder of the invention, the ingredient of the invention or the food product of the invention as a satiating product in humans or animals.

## BRIEF DESCRIPTION OF THE FIGURES

**Figure 1:** results of the DSC analysis of a collagen powder sample of the invention (1A), of a Collapro® sample (1B), of a Kapro® sample (1C) and of a commercial gelatin sample. The collagen powder sample of the invention (1A) contains an endothermic peak characteristic of native collagen, while the other samples do not have it, so they are not native collagen (1B, 1C and 1D).

**Figure 2:** RINDS after chemical treatment.

**Figure 3:** Microscope captures at different dilutions with detail of the different fiber hierarchies that microscopy observation allows to resolve.

**Figure 4:** images of the gels obtained in the Bloom test for the powder samples of the invention (P1 and P2), for Kapro C®, for Kapro SF® as well as for a gelatin sample.

**Figure 5:** image of the supernatant that appears in the gelling of one of the samples of the collagen powder of the invention.

**Figure 6:** Average weight in grams of the animals during the treatment with the different experimental groups during the treatment. Data are represented as group mean $\pm$ SEM.

**Figure 7:** Weight difference between the end and the beginning of the average treatment in grams. Interaction test ANOVA 2x2 $p < 0.005$; t-Student * $p < 0.0001$ HFSPF CAS vs HFSPFCOL, * $p < 0.0001$ PF CAS vs PF COL. Data are represented as group mean $\pm$ SEM.

**Figure 8:** Average intake of the different experimental groups expressed in g (A) and% with respect to the Control group (B). Effect of the interaction between the protocol and the type of supplemented protein on the intake (C). ## $p < 0.01$ vs HFS; ### $p < 0.001$ vs HFS; $p = 0.05$ vs Casein 2h. Note: Group 1 (Control) has not been considered in the statistical analysis, using Group 2 (HFS Control) as the reference group for 1-way ANOVAs.

**Figure 9:** comparison of serum ghrelin levels for the Collagen 2h versus Casein 2h group. The group treated with collagen produced a significant decrease in ghrelin levels at 2 hours compared to the group treated with casein.

## DETAILED DESCRIPTION OF THE INVENTION

In order to facilitate understanding and clarify the meaning of certain terms in the context of the present invention, the following definitions are provided:

**"Dry powder":** This is the powder of the invention obtained after a drying process to remove a large amount of water, until reaching a moisture level of between 1% and 18%.

**"Collagen":** it is a protein whose basic unit molecule called tropocollagen is made up of three chains of amino acids intertwined and linked together by hydrogen bonds. The tropocollagen units are grouped in different orders giving rise to fibers that in turn are grouped into fiber bundles and finally into large fiber bundles.

**"Satiating properties":** refers to the ability of the powder of the invention to satisfy the need for food or drink for a certain period of time.

**"Gelatin":** It is the product that is obtained after denaturation of collagen, usually by applying high temperatures for a certain time.

**"Native collagen":** refers to the property of a certain collagen to maintain its native state, that is, to maintain the structure that it naturally presents in the collagen source from which it comes, preferably the dermis. It can be said that the native state of collagen or native collagen is the opposite of denatured collagen.

**"Bloom degrees":** It is a measure that serves to characterize the gelling power of a product. It is the force that a cylinder needs to penetrate a 6.67% collagen gel in water until it reaches a depth of 4mm. It is a standard measure of the force applied to cause deformation at a standardized concentration and temperature in a gel. It is commonly used to characterize gelatins, which are marketed according to their capacity to gel. There is a correlation between the Bloom degree and the gelatin concentration. The greater the amount of gelatin in a product, the greater the gelling capacity and the higher its value in Bloom Degrees.

**"Undigested collagen fraction":** it refers to the remaining fraction of collagen that has not been digested by pepsin under certain conditions, particularly, within the context of the present invention, those of the pepsin powder digestion test.

**"Pepsin Powder Digestion Assay":** refers to an analytical test that allows determining the degree of resistance of a certain collagen to digestion under acidic conditions with the pepsin enzyme, a protease (endopeptidase) that acts on the peptide bond by breaking it, and thus releasing the amino group and the carboxyl group. This test is a simulation of digestion for 40 min, at a controlled temperature with the pepsin enzyme and hydrochloric acid that is intended to mimic the conditions of the stomach.

**"Percent swelling":** It is a measure that allows evaluating the increase in weight of collagen when it is subjected to the swelling test. The acid swelling test of powders consists of introducing the collagen powder for 2 hours under acidic conditions. After 2 hours a centrifugation is carried out, whereby the swollen collagen fibers are precipitated and weighed. By weight difference, the swelling in percentage is calculated.

**"Food or dietary supplement or ingredient":** In the context of the present invention it refers to any food component that incorporates the collagen powder of the invention and that is used in the manufacture or preparation of a food and continues to be present in the finished product, even in a modified form.

**"Food product":** In the context of the present invention it refers to all food that through chemical and/or physical industrial processes of treatment, handling, conservation and packaging is suitable for human consumption and can be sold to the public and contains the collagen powder of the invention in its composition or the supplement or ingredient of the invention.

**"Mechanical treatment":** In the context of the present invention, it is applied to the set of processes intended to extract and reduce the size and length of collagen fibers from the collagen sources used. Mechanical treatments involve chopping or grinding processes that are carried out in a controlled manner and always with temperature control to avoid the denaturation of collagen.

**"Collagen source":** refers to the raw material used for the extraction and subsequent treatment of collagen to give rise to the powder of the invention. In a preferred embodiment, the collagen source is of bovine, ovine, porcine, avian, fish origin, or a mixture thereof. In an even more preferred manner, the source of collagen is skins of bovine origin from animals between 0 and 3 years of age, preferably less than 2.5 years of age.

Collagen powder

The main aspect of the invention refers to a dry collagen powder with satiating properties characterized in that it comprises collagen with a high percentage of native collagen and very little or no gelatin.

More specifically, the invention refers to a dry collagen powder with satiating properties characterized in that:

a) more than 70% of the collagen is native;

b) it has a gelatin content of less than 4% by weight;

c) at least 95% of the powder has a granulometry of between 10 $\mu$m and 5 mm and a mean granulometry between 250 $\mu$m and 1 mm.

Preferably, the dry collagen powder consists of more than 75% native collagen, more preferably more than 80% and even more preferably more than 90%.

Also preferably, the dry collagen powder has a gelatin content of less than 2.5% by weight, preferably less than 1% by weight, and even more preferably in the absence of gelatin.

Gelatin can be isolated in a dry powder from the fraction soluble in ammonium sulfate at 10% concentration and its subsequent centrifugation at 18,000rpm. Using the Biuret method, the protein content of said fraction can subsequently be determined and expressed as a percentage. This fraction appears when collagen is degraded. The precipitation of native collagen using salts, leaving degraded collagen as the soluble fraction, including gelatin, was already described by Piez in 1967 (Soluble collagen and the components resulting from its denaturation, in: Treatise on Collagen (GNRamachandran, ed) Vol. 1, P.207, Academic Press, New York. We have found that when gelatin is added to a collagen sample, this method allows it to be accurately quantified.

The low percentage of this fraction or the absence of it is directly related to the fact that the collagen is native, that is, when the collagen has not been subjected to any denaturation or hydrolysis treatment and is not degraded. The fact that the percentage of gelatin is so low in the powder of the invention is also derived therefrom.

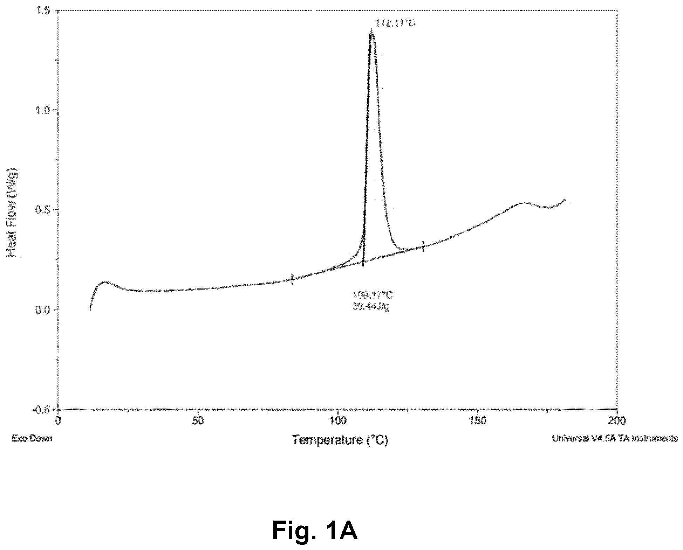

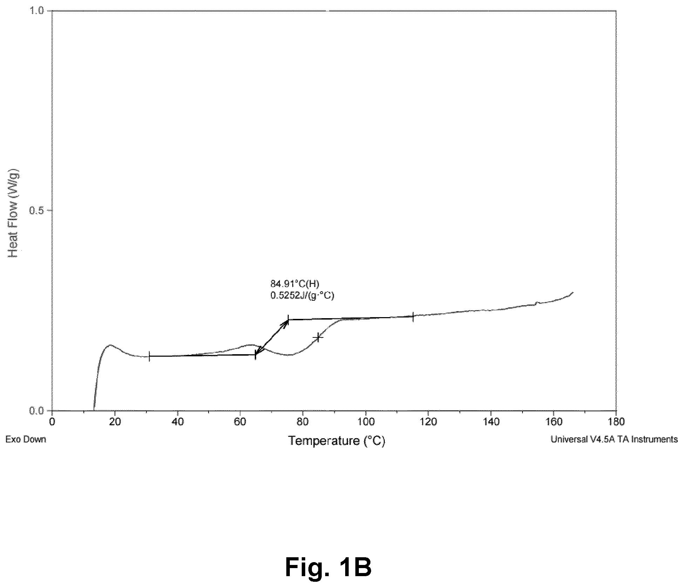

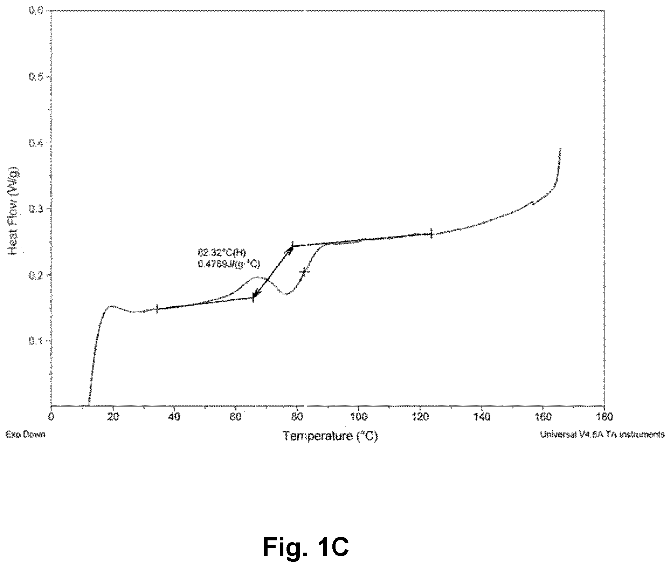

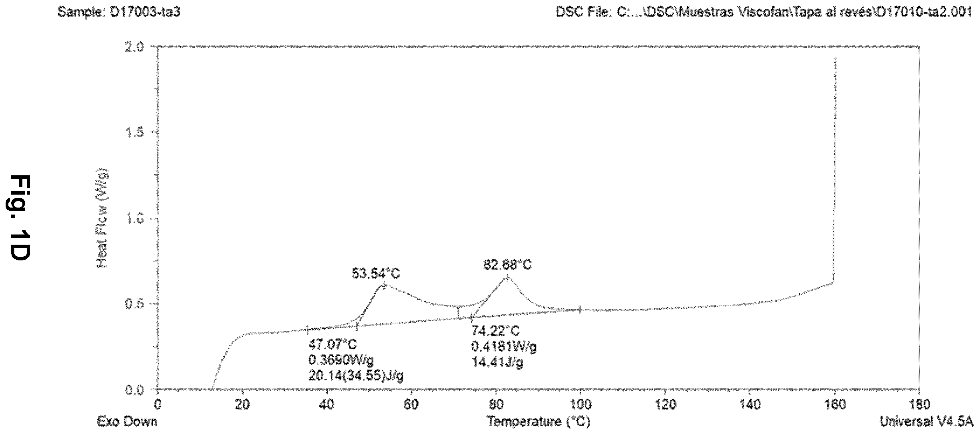

One way to easily measure the degree of denaturation of the collagen powder of the present invention is by DSC analysis. While the powder of the present invention presents a DSC profile of native collagen without detecting any denatured collagen and characterized by a marked endothermic peak around 109.67°C (see Figure 1A) and with a denaturation enthalpy of 46.65 J/g (sample moisture 15.5%), collagen powders from other previous commercial products such as Collapro® (see Figures 1B) and Kapro® (see Figure 1C) show a DSC profile without any endothermic peak characteristic of native collagen. Their profiles show peaks around 82°C, coinciding with one of the two peaks of gelatin, 82°C and 53°C (See Figure 1D). They are therefore powders with a lot of denatured collagen (gelatin) and little or undetectable native collagen.

Therefore, the powder of the present invention is made up of more than 70% native collagen (taking into account that a denaturation enthalpy of above 45 J/g °C is typical of native collagen and considering for calculations that an enthalpy 65 J/g °C for Type I bovine collagen represents 100% native collagen), preferably more than 75%, more preferably more than 80% and even more preferably more than 90%. Furthermore, the powder of the invention contains the fraction soluble in ammonium sulfate (% Gelatin), the most degraded collagen, always less than 4% by weight, more preferably less than 2.5% by weight, even more preferably less than 1% by weight, and preferably in the absence of the gelatin fraction.

Regarding the particle size, the collagen powder of the invention has a granulometry where at least 95% of the particles are between 10 $\mu$m and 5 mm, more preferably at least 90% of the particles are between 100 $\mu$m and 2 mm. The mean granulometry of the powder of the invention is between 250 $\mu$m and 1 mm.

On the other hand, the collagen powder of the invention has demonstrated a physicochemical behavior that is especially suitable for use as an additive with satiating properties in humans or animals.

On the one hand, it shows an extraordinary swelling capacity under pH conditions similar to those of the stomach. This swelling capacity is at least 32% higher than existing similar commercial products including that described in EP 2575496. In particular, the powder of the invention has a swelling percentage in the acid swelling test of collagen powders higher than 1500%, preferably more than 1550%. This extraordinary swelling capacity will favor the reduction of appetite or satiating effect once the powder of the invention reaches the stomach since, by increasing its volume in the acidic pH environment of the stomach, the cud will exert greater pressure on the gastric walls increasing the feeling of satiety.

Likewise, the collagen of the powder of the invention by maintaining a conformation that closely resembles the one that it has in the native state, with practically no denaturation and gelatin; it has a much higher resistance to enzymatic digestion. This allows the satiety effect to be much longer in time since the digestive system will need more time for its total degradation. The resistance to digestion of a collagen powder can be determined by the so-called pepsin digestion test. In this test, the undigested collagen fraction is measured after a simulation of digestion for 40 minutes, at a controlled temperature with the pepsin enzyme and hydrochloric acid, mimicking the conditions of the stomach. In this test, the powder of the present invention has an undigested collagen fraction after the pepsin powder digestion test greater than 30%. In the best case, the commercial products comparable to the powder of the invention do not show results of resistance to digestion with pepsin greater than 15.6%. That is, the powder of the present invention has at least twice the resistance to digestion of other commercial products with which it has been compared.

Likewise, there is a method to characterize the degree of gelling of a given collagen. The method is established in the ISO9665: 1998 (E) Standard and the Bloom degree or force that expresses the force that a cylinder needs to penetrate a 6.67% collagen gel in water until reaching a depth 4mm is measured. What is remarkable in relation to the collagen powder of the invention with respect to this test is not the Bloom value that it reaches, but rather that the gels prepared with the collagen powder of the invention for this test present a water supernatant that is not incorporated into the gel. This behavior probably has to do with the low percentage of gelatin present in the collagen powder of the invention, as well as the high resistance of collagen to gel under the test conditions (See Example 5). Gelatin has a great capacity to incorporate water but the absence of gelatin or its low percentage in the powder of the invention does not allow all the water to be incorporated and therefore the supernatant probably appears in the Bloom test. It should be remembered that the powder of the invention has a higher swelling capacity than other existing powders but when carried out in an acid medium, at pHs similar to those of the stomach.

The powder of the invention is in dry form and this implies that it has a moisture content of between 1 and 16%.

The dry collagen powder of the invention has a particle size of between 10 $\mu$m and 5 mm, preferably 80% of the particles are between 250 $\mu$m and 2000 $\mu$m. This favors, for example, the product having a suitable size for its intake, as well as for dispersing it in a matrix with which to combine it to be ingested.

As for the origin of collagen, it can come from any source of animal collagen. It is also preferable that the collagen source is as fresh as possible, so as to use the skins of recently slaughtered animals and which have not been salted and stored for a long time. In a particular embodiment, the collagen is of bovine, ovine, porcine, avian, fish origin or a mixture thereof. In a preferred way, the collagen is of bovine origin and from an animal between 0 and 3 years old, preferably less than 2.5 years old.

Dry collagen powder preparation method

An object of the present application is also a method for obtaining the collagen powder of the invention comprising:

a) Mechanical treatment of a collagen source, previously conditioned and acidified, to reduce the size of the fibers until a mass of collagen is obtained;
b) Successive dilutions of the collagen mass of step a) at a temperature below 25 ° C to obtain a concentrated collagen mass;
c) Homogenization of the mass at a temperature below 25 ° C and extrusion of the collagen mass of step b);
d) neutralization of the collagen mass of step c) with an alkaline agent to a pH of between 6 to 9;
e) Drying the collagen mass of step d) to a moisture of between 1% and 18%; and
f) Grinding the dry mass of step d) until obtaining a collagen powder.

The first step of the method has the purpose of reducing the size of the collagen fibers until obtaining a viscous and homogeneous collagen mass that will be processed in successive steps. For this, the previously conditioned and acidified source of collagen is mechanically treated. Any means capable of mechanically reducing the size of the fibers can be used in step a) of the method, although preferably the mechanical treatment is carried out by grinding until a homogeneous mass is obtained.

As a prior step to this first step of the method of the invention, the collagen must be conditioned and acidified. The conditioning and acidification treatment of the collagen source of step a) comprises:

i. chemically removing the hair from the collagen source if the source used comprises them,
ii. optionally, one or more washes with oxidizing agent,
iii. optionally, alkaline treatment to remove fat and other impurities from the collagen source; and
iv. acidification of the collagen source to a pH between 0.1 and 4.

Thus, the conditioning of the collagen source begins with the treatment of the collagen source, usually bovine, ovine or porcine skins that have hair and that must be removed. For this purpose, a chemical treatment with sulfuric salts such as $Na_2S$ and an alkaline treatment with $Ca(OH)_2$ is generally carried out.

Then, for proper conditioning, one or more washes with mild oxidizing agents such as dilute hydrogen peroxide can optionally be carried out to bleach and decontaminate animal skins by oxidation of the salt residues of the hair removal treatment.

Then, optionally, a treatment with an alkaline solution such as, for example, calcium hydroxide, sodium hydroxide or alkaline buffer systems with or without enzymes is carried out to soften, especially very compact skin and to remove traces of fat and other remains or impurities present in the treated skins.

Finally, once the skins have been conditioned, the collagen source is subjected to acidification for being processed in the method of the invention. For acidification, the skins are subjected to successive washes in acidic media, for example, with 33% HCl, to bring the pH to between 0.1 and 4. This pH is suitable for skin swelling as well as for transformation of this material and to make the fibers accessible in subsequent mechanical treatments.

After the mechanical treatment, step b) of the method of the invention is aimed at obtaining a mass, preferably with a percentage between 3 and 7% of collagen and reducing as much as possible or even completely eliminating the presence of gelatin. This step involves successive dilutions by grinding and subsequent homogenization under controlled conditions keeping the temperature below 25°C. The result of this step is a mass with a high percentage of non-denatured native collagen.

Then the mass is extruded through an extrusion head to produce a collagen film of variable dimensions depending on the collagen mass to be extruded. In a particular embodiment, for example, the film can have dimensions of 12 cm wide x 0.6mm thick. This produced film is processed in the following steps.

Since the extruded film is still at a strongly acidic pH, step d) comprises the neutralization of the film by an alkaline agent in solution to a pH between 6 and 9. Any suitable alkalizing agent can be used, either a strong base or a weak base. Neutralization is preferably carried out by means of a bath in a basic solution of sodium or potassium hydroxide. The ionic exchange between the collagen film and the solution takes time depending on the speed of diffusion. Normally, times between 1 and 60 minutes are sufficient for the neutralization of the collagen mass. Optionally, as a preparation for the drying step, the neutralized film can be washed once or several times.

Step e) comprises drying the neutralized film to a moisture of between 1 and 16%. It is important that drying is carried out at a low temperature to avoid possible denaturation of collagen. Drying can be carried out by different means, although ideally it is carried out in dryers by using forced air and at temperatures below 60°C until the product reaches said moisture, which can take from a few hours to 24 hours, depending on the capacity of the dryer.

Finally, in step f) after drying, the film is crushed or ground until obtaining the collagen powder of the invention. The grinding can be carried out by any method or means, although always avoiding that the temperature rises above the denaturation temperature in the process. Grinding is preferably carried out in a mincer with a temperature control so that it does not exceed 25 °C and even more preferably it is carried out by cryogrinding with the aid of dry ice or similar cryogenic substance. Cryogrinding has the advantage that it allows the glass transition temperature of collagen to be reached, making it a fragile material that is susceptible to being fragmented into small particles during the mincing process, but without denaturing it. The dry collagen powder of the invention has a granulometry (measured by a Malvern Instruments Mastersizer 3000 laser diffraction particle size analyzer) between 10 $\mu$m and 5 mm, and preferably with the 80% of particles between 250 and 2000 $\mu$m.

Food or dietary supplement or ingredient

Another aspect of the invention is a food supplement or ingredient comprising the collagen dry powder of the present invention.

The food supplement or ingredient in addition to containing the collagen powder, may contain other ingredients such as preservatives, antioxidants, colorants, flavorings or any other component commonly used in the food industry.

The supplement or ingredient of the present invention is intended to be incorporated into any food in order to increase its satiating power. In other words, the supplement of the invention may be useful in the context of a dietary treatment to reduce appetite and lose weight for merely aesthetic purposes, but it may also be useful in medical treatments to combat obesity and insulin resistance in pre-diabetic or diabetic patients.

On the other hand, the supplement of the invention is both applicable in human food and in animal feed.

Food product

Somehow related to the previous aspect, a food product comprising the dry collagen powder of the invention or the supplement or ingredient of the invention is also an object of the present invention.

The food product can be both a processed and an unprocessed product to which the powder or the supplement or ingredient of the invention has been incorporated. In a particular way, and without claiming to be an exhaustive list, the food product can be a meat product, a dairy product, a pasta, an egg product, a fish derivative, etc

Use of the dry collagen powder, the supplement or ingredient and the food product of the invention

A final object of the invention is the use of the dry collagen powder of the invention, as well as the ingredient and the food product of the invention.

The powder of the invention and products derived from it, can be used as a satiating product.

The powder of the invention, the supplement or ingredient or the food product of the invention have a use as a satiating agent in humans or animals for the reduction of appetite and with the ultimate goal of weight loss, as long as there are no pathological processes that by themselves may also cause weight loss. This use could be considered as a dietary or aesthetic use.

The way in which the powder or the additive of the invention can be used is by supplementation in any of the meals of the day, be it breakfast, lunch or dinner, although preferably at breakfast.

It can also be incorporated during the manufacturing process of processed foods to increase the satiating power of them and giving rise to a food product according to the present invention.

EXAMPLES

Below, the way in which the preparation of the powder of the invention can be carried out is illustrated by means of some examples, as well as some examples where the powder is characterized by means of different parameters and these parameters are compared with other commercial collagen powders. In vivo examples on rats are also provided which demonstrate the weight loss effect caused by the collagen powder of the invention, as well as the satiating effect. These examples are presented by way of demonstration, but are not intended to limit the invention in any way.

## Example 1: Preparation of collagen powder

The reference raw material used for the preparation of the powder was skin of bovine origin. This raw material is well known, as are the collagen extraction methods, which is why it is considered an ideal material for the production of native collagen powder with satiating potential.

From the skins of young calves (approximately 2 years old) the inner layer of the skin called the chorion was separated from other layers that are not of interest such as the grain, meat and fat layer. This chorion layer is rich in collagen protein, specifically more than 80% of the total protein is collagen protein and is what is known as Split.

Once the collagen-rich part, the Split, had been obtained, the chemical treatment of purification and extraction of collagen was started, which consisted of the following steps:

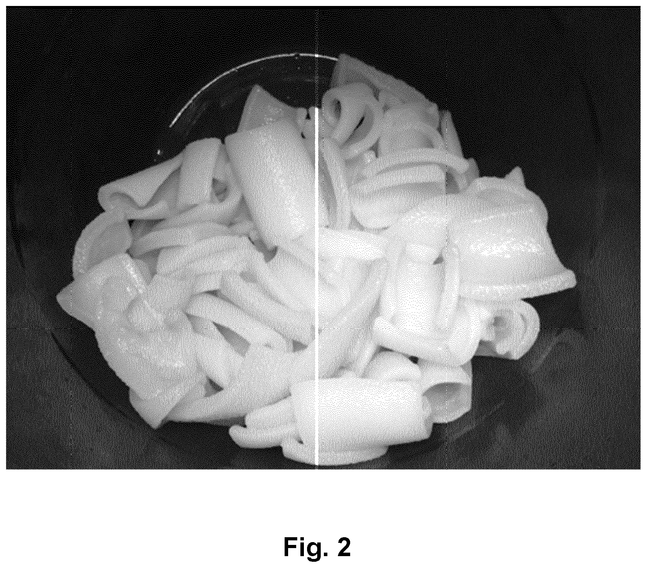

1. A pretreatment with hydrogen peroxide was carried out, which produced the oxidation of the residual salts so that they can be solubilized and subsequently eliminated by washing. For this purpose, raw material was added together with the 35% hydrogen peroxide to a final concentration of 0.58% (% V/W); and mixed for 1 hour at room temperature. Once the hydrogen peroxide treatment was finished, the liquid was drained and 2 washes of 30 minutes each were carried out with water.
2. Subsequently, a controlled acidification was carried out with hydrochloric acid until reaching a final concentration of 13.85% (% V/W), and it was left to work at least overnight at room temperature. After this step, a washing with water was carried out, to eliminate the excess acid from the previous step, thus obtaining the "Rinds" (see Figure 2).

From these Rinds, the native collagen fibers were extracted by means of a mechanical mincing process in two phases; coarse mincing and fine mincing, using a meat grinder (Gesame GP32). In this way, a heterogeneous paste with a high solid content was obtained. To carry out the transformation of this collagen paste into films of adequate quality, a step of kneading and diluting the mass was necessary. Therefore, the collagen paste was mixed with the necessary amount of water and/or ice in a Z-blender to a final percentage of solids between 4 and 7%. From this diluted mass, the collagen films were made at room temperature, using a laminator (Vansda, Thermal Laminator). The films were subjected to a dilute ammonium hydroxide bath (0.03%) and washed with water. Finally, these wet films were allowed to dry at room temperature.

Finally, the dried films were minced to obtain a powder with an average particle size of less than one millimeter.

To this end, cryogrinding of the collagen films was carried out to obtain a fine particle with a flake appearance. For this cryogrinding process, a kitchen robot (Robot Coupé) with a toothed blade was used. The film samples were introduced together with dry ice in a 1: 3 ratio, and the material was grinded at maximum speed for 5 minutes.

Thanks to the use of dry ice, it was possible to reach the glass transition temperature of the previously described collagen, making it a fragile material that could be fragmented into small particles during the mincing process without denaturing it. Thus, a collagen powder is obtained with an average particle size between 250 and 1000 $\mu$m.

The analysis of the samples obtained was carried out both on the intermediate product and on the final product. The following parameters were analyzed, obtaining the results of table 1:

**Table 1 Values of Moisture, pH, acid swelling of the powder of the invention of example 1**

|  | Collagen powder |
|---|---|
| % Moisture | 13.7% |
| pH | 7.6 |
| % acid swelling | 1620% |

**Example 2: Observation of collagen fibers by optical microscopy**

Known quantities of the collagen powder samples of the invention obtained according to example 1 have been dispersed in 20mM HCl so as to obtain a collagen concentration of 0.8%. This dispersion has been made with the help of ULTRA-TURRAX®. The powder has been added little by little while stirring for about 5 minutes. Once the mixture is generated, it is left for 24 hours at 20°C.

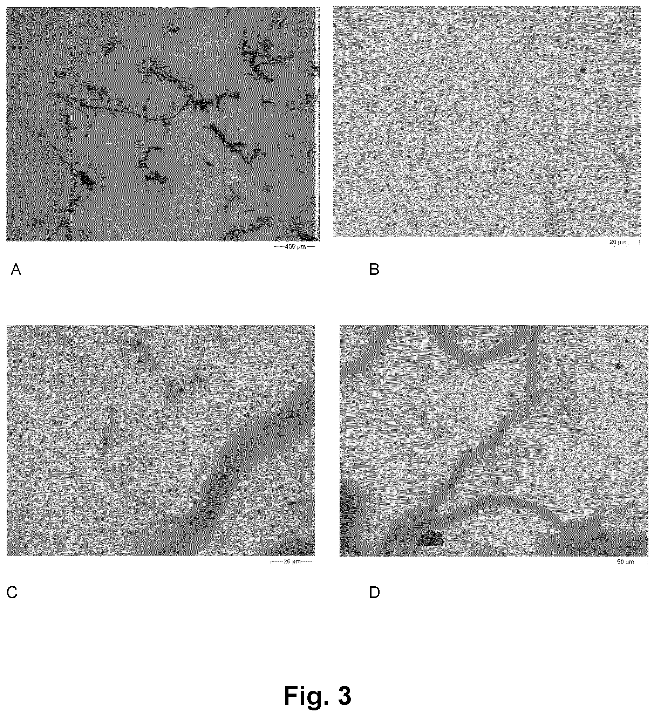

The following day the mixture obtained is processed to obtain different dilutions (between 1:10 and 1: 100) and small fractions of the prepared dilutions are stained with the Sirius Red dye. In Figure 3 the captures of microscope at different dilutions can be seen in great detail, wherein collagen fibers of different sizes and with different degrees of aggregation are shown. Fibrils of very small diameters (3B) to fibers of more than 20 $\mu$m (3C) and more than 1 mm in length (3A) can be observed.

**Example 3: ammonium sulfate-soluble fraction**

A quick way to quantify the degree of degradation and hydrolysis of native collagen is to determine the fraction soluble in 10% ammonium sulfate after centrifuging the collagen powder previously resuspended in 25mM HCl at 18,000rpm. Said fraction, which contains gelatin, appears especially in deteriorated collagens.

Subsequently, the collagen protein content in said fraction is quantified with the Biuret method and the result is expressed as a percentage.

Table 2 shows the soluble fraction (in percentage) of the collagen powder samples of the invention (P1 and P2) in comparison with other commercial collagen powders:

**Table 2 Percentage of the soluble fraction of two lots of powder of the invention (P1 and P2) and of other commercial collagen powders**

|  | P1 | P2 | Kapro C® | Kapro SF® | Collapro® fine powder |
|---|---|---|---|---|---|
| % G | 0.0 | 2.0 | 14.5 | 20.2 | 18.1 |

**Example 4: DSC analysis to determine the native state of collagen**

To analyze the native state of collagen, a DSC analysis of a sample of the powder of the invention and of two other samples of commercial collagen powders (Collapro ® and Kapro ®) was used.

Samples of the order of 3-25mg, depending on the water content, were weighed and encapsulated in hermetic aluminum crucibles to avoid evaporation of the water. The sealed crucibles with the samples were weighed.

In a DSC equipment the scan was made at a speed of 10 ° C/min. The crucibles were reweighed after the scan to verify that they had not lost sample or water. The scan was started at a temperature of at least 25 ° C lower than that expected for the first transition (denaturation for example), in this case at 15 ° C. For the analysis of the thermogram applying the software and its interpretation, the keys in collagen are: denaturation temperature (triple helix stability), denaturation enthalpy (amount of crystalline, ordered, native collagen), temperature and size of the Tg (temperature of glass transition) and the shape of the thermogram. Collagen denaturation is an endothermic process, that is, a more or less wide peak appears above the baseline. The temperature at which this peak is observed will depend on the water content of the collagen sample, being 110-120°C for collagen with a low percentage of water.

In Figures 1A-C, which show the result of the DSC analysis, it is observed how the powder of the invention presents a characteristic peak of a native collagen (Figure 1A) while the samples of Collapro ® (Figure 1B) and Kapro® (Figure 1C ) show a clear profile of denatured collagen without any characteristic endothermic peak of native collagen. Their profiles show peaks around 82°C, as in the case of gelatin, which also has a peak at 82°C, in addition to another at 53°C (See Figure 1D). Therefore, the samples 1B and 1C are powders with a lot of denatured collagen (gelatin) and little native collagen.

**Example 5: Bloom test (ISO9665 standard)**

To observe the differences between the different powdered collagen samples, gel strength measurements are carried out through the Bloom degree test. This test makes it possible to analyze the way in which different collagen samples gel.

Therefore, 7.5 grams of each sample to be tested were dissolved in 105 mL of distilled water, which corresponds to a concentration of 6.67%, by stirring with a glass rod. Next, to improve the uniformity of the samples and prior to heating to 65 ° C, all the dispersions of the different collagen powders were subjected to homogenization in Ultra-Turrax at 15000 rpm for 1 min.

After homogenizing the dispersions in the Ultra-Turrax, they are heated in a water bath at $65 \pm 1 °C$ for 15 min, gently stirring each dispersion a couple of times during this time with a glass rod.

Next, the containers are covered with parafilm (jar with an internal diameter of $59 \pm 1$ mm and 85 mm high, specific for the measurement of Bloom degrees), and they are allowed to stand at room temperature for 1 h. Subsequently, they are placed in the bath with water at $10.0 \pm 1 °C$ for $17 \pm 1$ h. After 17 h, the jars with the gelatins already formed are removed from the water bath, and they are warmed up to room temperature for 1 h. Finally, the measurements of each sample are carried out in the texture analyzer at 20 °C.

The measurement is made by penetrating a cylinder (0.5 "Radius Cylinder Probe P/0.5R) into the gelatin contained in the jar, using the 5 kg cell and at a deformation rate of 0.5 mm/s according to the ISO Standard.

Once a force of 4 g has been reached, the cylinder penetrates the gel to a depth of 4 mm, and the force necessary to reach this depth is the so-called 'Bloom Strength' (g) of the gel.

The results for the different samples tested are shown in Table 3:

Table 3. Bloom degrees of two lots of powder of the invention (P1 and P2), Kapro C ® and Kapro SF ® and a commercial gelatin

|  | P1 | P2 | Kapro C® | Kapro SF® | Gelatin |
|---|---|---|---|---|---|
| **Bloom degrees** | 43 | 45 | 112 | 67 | 108 |

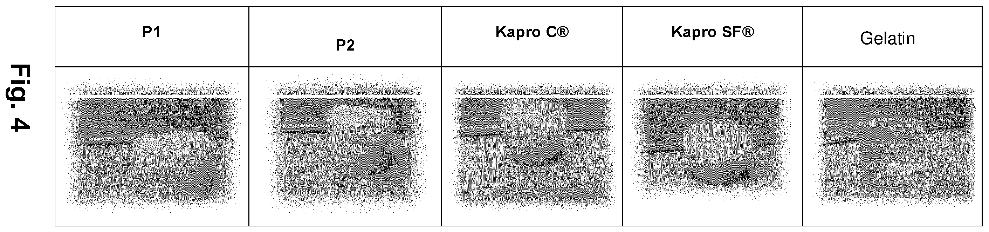

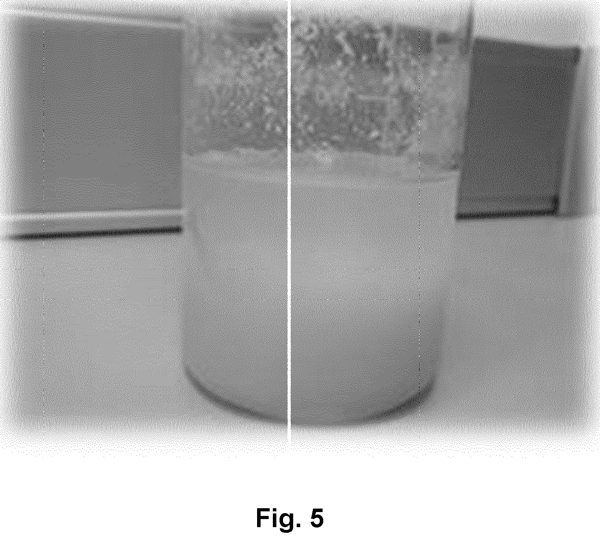

Figure 4 shows the gels obtained for each of the samples tested. In relation to the samples of the powders of the invention P1 and P2, it should be noted that once they gelled, the samples showed a liquid supernatant approximately 1 cm thick (see Figure 5). To perform the Bloom test measurement, it was necessary to remove said supernatant since the test does not begin until the plunger does not reach a minimum resistance of 4g and the water does not offer that resistance. The low gelling capacity of the powder of the invention is therefore noteworthy. Even though the sample is heated at 65°C for 15 minutes during the analytical process, it appears that it does not produce a complete gelatinization effect of the collagen of the powder of the invention, probably due to the high resistance to denaturation of the collagen of the invention, which once again confirms its proximity to collagen in its native state.

## Example 6: Pepsin Digestion Test

The pepsin digestion test allows the digestion conditions in the stomach to be simulated and allows the degree or percentage of undigested collagen to be determined at the end of the test.

Selective enzymatic hydrolysis of collagen will give rise to intermediate species such as peptones, peptides, and even amino acids.

The pepsin enzyme is a protease (endopeptidase) that acts on the peptide bond breaking it, thus releasing the amino group and the carboxyl group.

Two samples according to the invention (P1 and P2) as well as samples of Kapro C®, Kapro SF® and Collapro® were tested. 0.6 grams per sample were weighed.

The "gastric simulant" pepsin solution was prepared at a concentration of 3.2 g/L and left for 15 minutes for the activation of the pepsin. 40 ml was added for the 0.6 g of each sample and was left to work at 37 ° C for 40 minutes.

The reaction was quenched with 1N NaOH. It was filtered through 5-9 micron filters to obtain the undigested solids on the filter and dried at 160 ° C for 1 hour in a vacuum oven.

The results (see table 4) were expressed as a percentage of undigested collagen versus initial collagen in dry extract. For this purpose, the following calculations were carried out: In this technique, the percentage of moisture in the collagen powders must be taken into account.

- *Calculation of the initial collagen dry weight:*

$$\textit{Initial collagen dry weigh } (Psi) = Pcol - (P col \times \% \ moisture/100)$$

- *Calculation of% undigested solids:*

$$\textit{Psf} = Ps - Pp - Pf$$

$$\% \ Undigested \ solids = (Psf \times 100)/Psi$$

wherein:

Psi = Initial collagen dry weigh
Pcol = collagen sample weight (g)
Psf = final solid dry weight (g)
Ps = dry weight (g)
Pp = plate weight (g)
Pf = filter weight (g)

**Table 4 Percentage of undigested collagen of two lots of powder of the invention (P1 and P2), Kapro C ® and Kapro SF ® and Collapro ®**

|  | P1 | P2 | Kapro C® | Kapro SF® | Collapro ® fine powder |
|---|---|---|---|---|---|
| % Undigested | 37 | 42 | 10 | 2 | 0 |

A greater resistance to enzymatic digestion of the powder of the invention is clearly observed compared to other commercial products, which in some cases is completely digested. The increased resistance to enzymatic degradation in this "gastric simulant" is believed to cause a delay in digestion and thus increase the satiating effect of the product.

**Example 7: Acid swelling test**

Swelling (SW) measures the water retention capacity of collagen in an acid medium under pH conditions similar to those of the stomach. Two samples of the powder of the invention (P1 and P2) as well as three samples of commercial collagen powders (Kapro C®, Kapro SF® and Collapro®) were tested.
For this purpose, 0.6g of collagen powder from each sample was weighed into a falcon tube suitable for a centrifuge and 30ml of 0.1N HCl were added. After stirring, it was allowed to stand for 2h at room temperature. It was centrifuged for 10 minutes at 18000 rpm at 23 ° C.
The supernatant was removed and the pellet was collected for immediate weighing. The sample was allowed to dry in an oven at 160 ° C to constant weight.
The% swelling was calculated using the formula:

$$\% \ SW = ((PH/PS) \times 100) - 100$$

Wherein:

· PH: wet weight
· PS: dry weight

The results of the swelling test are shown in table 5:

**Table 5 SW swelling results (%) of two lots of powder of the invention (P1 and P2) as well as two samples of commercial collagen powders (Kapro SF® and Collapro®).**

|  | P1 | P2 | Kapro SF® | Collapro ® fine powder |
|---|---|---|---|---|
| % SW | 1978 | 1842 | 1468 | 1051 |

These results demonstrate that the collagen powder samples of the invention have a significantly higher water retention capacity than commercial products. This will directly affect the satiating power of the powder of the invention, which will be greater than that of other commercial powders.

**Example 8: In vivo test on weight loss**

In this test, a lot of collagen powder equivalent to the P1 and P2 of the invention was evaluated, selected for its possible effects on weight loss in an in vivo model with rats that had previously developed obesity through the ingestion of a high fat and sugar diet (HFS).

The proposed work scheme for this test was divided into 3 steps. The first step was a 4-week acclimatization of the animal model used, Wistar rats with an approximate weight of 50-75 grams, to which a control diet was administered (Envigo, diet 2014S).

The second step began when the animals had reached an average of 200 grams and lasted 8 weeks. It consisted of the administration of a powdered diet high in fats and sugars (Research Diets, diet 12451) with the aim of inducing a dietary obesity model, with the exception of the control group that remained on a powdered control diet.

Finally, the third step was treatment/intervention and consisted of administering the following ad libitum diets to the experimental groups (throughout the day): control diet; high fat and sugar protein free diet (HFSPF); and protein-free diet (PF). The last two were supplemented with protein in 14.3% M/M of the total weight of the diet. In the case of the casein groups, the diet was supplemented with 14.3% casein and in the case of the collagen groups with 10% collagen plus 4.3% casein until adding 14.3% protein. The percentages of supplementation of the diets were chosen based on the literature studied and the previous pilot tests.

The experimental groups with collagen were also supplemented with tryptophan until equalizing the amount of this amino acid in the casein group, given the absence of this essential amino acid in collagen (Table 6).

**Table 6. Percentage of essential amino acids (EA) in the control and treatment diets. Tryptophan was supplemented based on the amount contained in the casein-only group. The EA data were extracted from the composition provided by the manufacturer of the control diet. For the casein and collagen AE data, a mean estimate was made of the information of various marketed products.**

| Essential amino acids (AE) | AE Control | AE Casein | % AE With casein | AE Collagen | % AE With collagen | % total AE Casein (4.3%) + Collagen 10% diet | Amount supplemented in collagen diet |
|---|---|---|---|---|---|---|---|
| Threonine | 0,50% | 4.40% | 0.15% | 1.72% | 1.20% | 1.35% | No |
| Leucine | 1.40% | 8.30% | 0.42% | 2.62% | 1.83% | 2.25% | No |
| Isoleucine | 0.60% | 5.50% | 0.18% | 1.36% | 0.95% | 1.13% | No |
| Valine | 0.70% | 6.50% | 0.21% | 2.17% | 1.52% | 1.73% | No |
| Phenylalanine | 0.70% | 4.50% | 0.21% | 1.90% | 1.33% | 1.54% | No |
| Methionine | 0.30% | 2.50% | 0.09% | 0.54% | 0.38% | 0.47% | No |
| Lysine | 0.70% | 7.40% | 0.21% | 3.07% | 2.15% | 2.36% | No |
| Histidine | 0.40% | 2.80% | 0.12% | 0.72% | 0.50% | 0.62% | No |
| Trypophan | 0.20% | 1.30% | 0.06% | 0.00% | 0.00% | 0.06% | 0.14% |

After the treatment, the animals were slaughtered and different tissues were extracted for later analysis.

The work was carried out for two consecutive weeks, in which the intake and weight of the animals was controlled every 2-3 days. Thus, a total of **55 animals** arranged in individual cages were used, which were randomized into the five experimental groups described below (n = 11 rats/group):

- **Group 1: Control group (CT).** Administration of diet 2014S (14.3% M/M protein), ad libitum throughout the day.
- **Group 2: High fat and sugar protein free + casein diet group (control protein; HFSPF CAS).** Administration of diet D12451px07 from Research Diets supplemented with casein in an amount equivalent to 14.3% in dry weight of the total daily diet.
- **Group 3: High fat and sugar protein free + collagen diet group. HFSPF COL.** Administration of diet D12451px07 from Research Diets supplemented with collagen in an amount equivalent to 10% in dry weight of the total daily diet, plus 4.3% of casein in order to equal the protein intake to that of the control.
- **Group 4: Protein free + casein diet group (control protein, PF CAS).** Administration of Envigo 93328 diet supplemented with casein in an amount equivalent to 14.3% in dry weight of the total daily diet.
- **Group 5: Protein free + collagen diet group (PF COL).** Administration of Envigo 93328 diet supplemented with collagen in an amount equivalent to 10% in dry weight of the total daily diet, plus 4.3% of casein in order to equal the protein intake to that of the control.

RESULTS: WEIGHT ASSESSMENT.

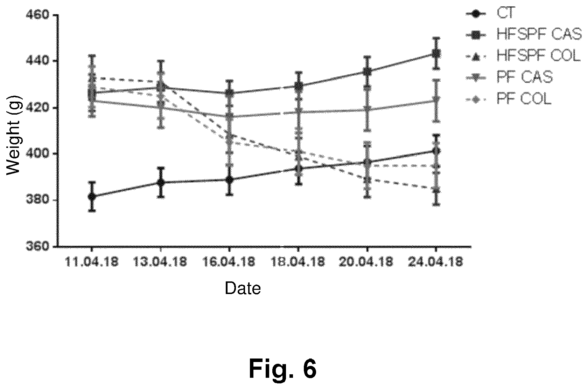

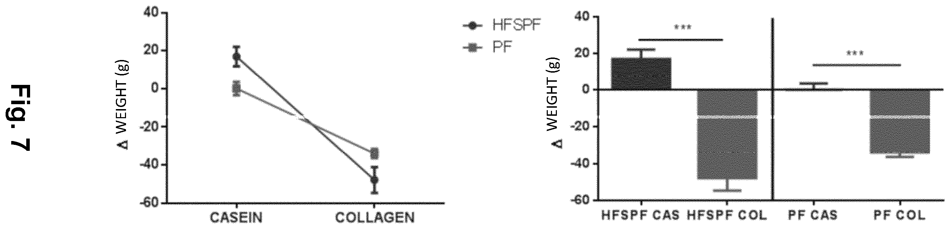

From the third day of treatment, a particularly drastic decrease in the weight of the rats was observed in the groups supplemented with collagen both in the HFSPF diet and in the PF diet (Figures 6 and 7). In fact, the weight loss in the groups supplemented with collagen resulted in them having a similar weight to that of the control group at the end of the study (Figure 6). On the other hand, in the groups supplemented with casein, no large changes in weight were observed (Figures 6 and 7).

To rule out that this weight loss was due to dehydration, since the undigested collagen could be retaining water, the water consumption of the animals was measured. However, no significant differences were observed between the different experimental groups. So dehydration was ruled out as a cause of weight loss. In addition, the animals did not show external signs of dehydration, nor any other apparent alteration.

EVALUATION OF THE WEIGHT OF ORGANS AND TISSUES

During the slaughter of the animals, different organs were studied at macroscopic level, which were extracted and weighed for later analysis. In addition, samples of several specimens were collected in order to perform a toxicological analysis.

Thus, different types of fatty tissues were collected that were referred as epididymal, mesenteric, brown, retroperitoneal and subcutaneous fat regarding the weight of the animal. As shown in Table 7, all fatty deposits (except brown) showed significant differences with the treatment, with a lower percentage of these tissues with respect to the weight of the animal in the collagen groups than in the casein groups. Thus, it can be concluded that the collagen treatment significantly decreased the percentage of fat mass, affecting practically all the adipose deposits of the animals.

**Table 7. Weight in grams of the organs and tissues with respect to the weight of the animal of the experimental groups supplemented with collagen and casein with the HFSPF and PF diets. ANOVA 2x2 test. Data are represented as group mean $\pm$ SEM.**

|  | HFSPF CAS | HFSPF COL | PF CAS | PF COL | Diet | Treatment | Interaction |
|---|---|---|---|---|---|---|---|
| EPIDIDYMAL FAT | 13.30 ± 0.91 | 10.42 ± 1.24 | 12.03 ± 0.84 | 10.57 ± 0.66 |  | 0.0254 |  |
| MESENTERIC FAT | 5.815 ± 0.551 | 4.562 ± 0.321 | 4.849 ± 0.321 | 4.364 ± 0.392 |  | 0.0389 |  |
| BROWN FAT | 0.760 ± 0.038 | 0.792 ± 0.067 | 0.745 ± 0.61 | 0.884 ± 0.078 |  |  |  |
| RETRO G. | 15.52 ± 1.15 | 11.48 ± 1.06 | 14.26 ± 0.67 | 11.07 ± 0.87 |  | 0.0005 |  |
| SUBCUTANEO US FAT | 7.967 ± 0.737 | 5.821 ± 0.517 | 7.678 ± 0.403 | 5.546 ± 0.481 |  | 0.0004 |  |
| VISCERAL FAT | 34.64 ± 2.45 | 26.46 ± 2.54 | 31.14 ± 1.58 | 26.01 ± 1.83 |  | 0.0034 |  |
| TOTAL FAT | 42.60 ± 3.07 | 32.28 ± 2.99 | 38.81 ± 1.96 | 31.55 ± 2.23 |  | 0.0017 |  |
| SOLEUS | 0.151 ± 0.011 | 0.158 ± 0.010 | 0.1573 ± 0.011 | 0.171 ± 0.007 |  |  |  |
| GASTRO | 2.686 ± 0.112 | 2.373 ± 0.085 | 2.324 ± 0.025 | 2.447 ± 0.104 |  |  | 0.0204 |
| LIVER | 9.550 ± 0.185 | 9.230 ± 0.312 | 9.674 ± 0.383 | 10.89 ± 0.569 |  |  |  |
| SPLEEN | 0.661 ± 0.036 | 0.534 ± 0.019 | 0.656 ± 0.038 | 0.704 ± 0.021 |  |  | 0.0060 |
| KIDNEY | 2.270 ± 0.078 | 2.160 ± 0.045 | 2.237 ± 0.062 | 2.356 ± 0.035 |  |  | 0.0235 |

When studying visceral fat (sum of all the deposits located in the abdominal cavity) and total fat (the former plus subcutaneous), both in percentage with respect to weight and in total grams, a decrease was also observed in those supplemented with collagen. **Therefore, it is concluded that the weight loss in the groups supplemented with collagen was basically due to the loss of fat.**

FINAL CONCLUSION:

Supplementation with 10% collagen in the diet induced a decrease in body weight that is attributable to a decrease in fat mass, without affecting muscle mass or observing symptoms of dehydration or gastrointestinal alterations, nor macroscopic alterations of toxic effects.

**Example 9: In vivo test on the satiating effect**

The capacity as a satiating agent of the collagen powder of the invention was evaluated, as well as its possible metabolic effects in an in vivo model with rats that had previously developed obesity by ingesting a high fat and sugar (HFS) diet.

Wistar rats with an approximate initial weight of 50-75 grams were used. The proposed work scheme for this trial was divided into 3 steps:

The **first step,** acclimatization, lasted for 3 weeks after receiving the animals included in the study, during which a control diet was administered (Envigo, diet 2014S)

The **second step,** induction of the obesity model, began when the animals had reached an average weight of approximately 180 grams and lasted 7 weeks. It consisted of the administration of a high fat and sugar diet (HFS-Research Diets, diet 12451) with the aim of inducing obesity and insulin resistance in the animals, except for the control group (group 1) that remained on the control diet. This group served as a control for the obesity model used.

Finally, when the animals fed with the HFS diet showed significant differences in weight and body composition with respect to the control group, the third step of the study began, which consisted of the treatment/intervention phase. During this phase, all the animals received a control diet and two clearly differentiated experimental strategies were carried out in parallel, to evaluate the potential satiating effect of collagen:

◦ The first strategy consisted in administering diets ad libitum to the experimental groups (throughout the day). The normal diet of the rats was supplemented with an amount of collagen/casein equivalent to 7.5% in dry weight of the total diet. Together with the diet, the animals had free access to water to promote the increase in the volume of collagen and, in this way, promote the satiating effect due to a greater gastric occupation.

◦ For the second experimental strategy, after a fasting period (o/n) of 12 hours, 4 g of diet containing the satiating agent or its control (e.g. pulverized collagen or casein equivalent to 7.5% w/w of the total daily intake), and the rat was allowed to feed for a period of 1.5 h to ingest as much as possible. After this time, and once the intake of the satiating agent was confirmed, diet was given ad libitum for the next 10.5 hours. During all this time the animals had free access to water. Once the intake was evaluated, the food and water were withdrawn for 12 hours (o/n) to keep the animals under fasting conditions again until the next day, in which the procedure was repeated.

Following these two experimental strategies and including the corresponding control groups (groups 1 and 2), a total of 60 Wistar rats arranged in individual cages were used, which were distributed into 6 groups (10 animals per group) according to the following scheme developed over seven consecutive weeks, in which the intake was controlled (daily).

- **Group 1:** Control diet group. Administration of diet 2014S of Envigo ad libitum throughout the experiment.
- **Group 2:** Obese control group. HFS diet (diet D12451, high in fat and sucrose) ad libitum for 7 weeks and Control diet (2014S) for 7 weeks thereafter.
- **Group 3:** Group treated with casein 24h (Casein 24h). 7 weeks of HFS diet (D12451). Then, Control diet (2014S) supplemented with casein equivalent to 7.5% w/w of the total daily intake for another 7 weeks.
- **Group 4:** Group treated with collagen 24h (Collagen 24h). 7 weeks of HFS diet (D12451). Then, Control diet (2014S) supplemented with collagen equivalent to 7.5% w / w of the total daily intake for another 7 weeks. 3/11

**Group 5:** Group treated with casein only in the morning (Casein 2h). 7 weeks of HFS diet (D12451). Then, Control diet supplemented with casein (equivalent to 7.5% w / w of the total intake but only between 8 and 9:30 am). The rest of the day (9:30 am - 8:00 pm) control diet (2014S) ad libitum. Fasting "overnight" and the next day this protocol is repeated.

- **Group 6:** Group treated with collagen only in the morning (Collagen 2h). 7 weeks of HFS diet (D12451). Afterwards, Control diet supplemented with collagen (equivalent to 7.5% w / w of the total intake but only between 8 and 9.30 am). The rest of the day (9:30 am - 8:00 pm) control diet (2014S) ad libitum. Fasting "overnight" and the next day this protocol is repeated.

RESULTS: FOOD INTAKE ASSESSMENT.

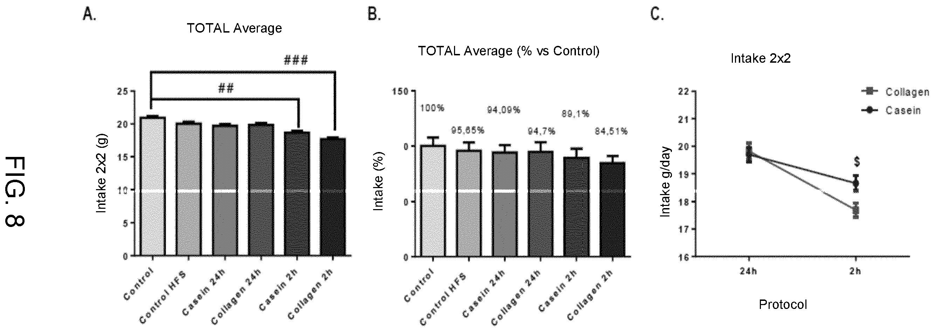

Individualized intake data for each of the 60 rats included in the study were recorded daily throughout the test. During the 7 weeks of study intervention, the intake of the different groups ranged from approximately 17 g to 21 g per day. The intake trend between the groups remained more or less constant throughout the 7 weeks, being the group that received the diet supplemented with collagen 7.5% w/w early in the morning (group 6) the one that presented values of lower intakes. Significant differences in intake were observed between the different groups when analyzing the data using an ANOVA analysis that compared all the groups with each other. Specifically, the groups that followed a feeding pattern with supplementation of the diet with protein at first hour of the day (groups 5 and 6) ingested less amount of diet than the rest of the groups (Figure 8B).

Especially interesting is the analysis by Anova 2x2 of the intake data of groups 3-6 (Casein 24h, Collagen 24h, Casein 2h and Collagen 2h). This analysis allows us to observe the effect of the two main factors; the type of treatment and the type of protein that the animals received. Statistical analysis of the data represented in Figure 8C indicates that there is a significant interaction between protocol and protein factors. The interaction graph shows that only in the groups that followed the 2-hour protocol (treatment intake early in the morning), the intake was higher when the protein was casein than when it was collagen. The analyzes that allow us to conclude about the simple effects confirm this hypothesis and significant differences in intake are obtained between the Casein 2h group and the Collagen 2h group when the animals followed the diet supplementation protocol early in the morning. This result supports the hypothesis of the potential satiating effect of collagen when administered early in the morning and is in line with previous results in which it was observed that this group 6 is also the one with the lowest weight gain.

Once the differences in intake were observed as a function of the protein supplemented in the diet when the rats received the feeding pattern with the supplementation early in the morning, the next objective was to try to elucidate the possible molecular mechanisms underlying the satiating effect of the collagen being observed.

To this end, blood samples were obtained from the tail of the animals belonging to groups 5 and 6 (Collagen 2h and Casein 2h) under fasting conditions, after ingestion of the diet supplemented with protein, and 3 hours after ingestion of the supplemented diet. Subsequently, the samples were processed and the plasma levels of ghrelin (orexigenic hormone that promotes ingestion) were analyzed.

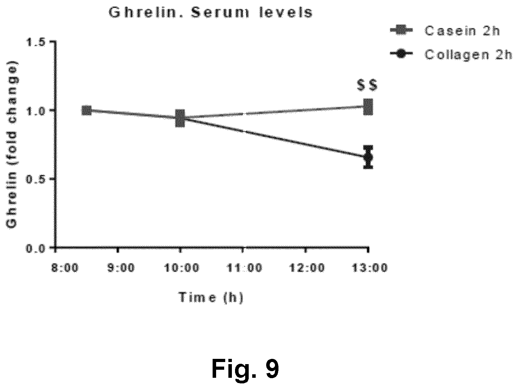

It is especially interesting to see that serum ghrelin levels decreased significantly in the Collagen 2h group in the hours after ingestion of the treatment compared to the Casein 2h group (see Figure 9).

EVALUATION OF BIOCHEMICAL PARAMETERS.

In the slaughter of animals, serum and plasma samples were obtained for the subsequent analysis of the following biochemical parameters of interest (Table 9). These blood samples were collected after a 12-hour fast from the rats. As can be seen in Table 9, no relevant changes were observed at the level of general metabolism of the animals as a function of the type of protein with which the diet was supplemented, when the animals followed the same feeding pattern. Statistically significant differences were only observed in the levels of HDL-Cholesterol and Cholesterol that were decreased in the Collagen 2h group compared to the Casein 2h group. However, when analyzing the Total Cholesterol / HDL ratio, these differences were neutralized, so this result has no special physiological relevance.

Furthermore, it should be noted that no abnormal values of transaminases (ALT and AST) were observed in any of the study groups, which makes it possible to rule out any toxicological effect.

The analysis by Anova 2x2 of the different parameters analyzed, including the protocol and the type of protein that the animals had received as main factors, it was observed that only for the Cholesterol, HDL-Col, triglycerides and insulin markers, there were significant differences depending on the protocol they had followed. The type of protein that the animals received was a determining factor in terms of ALT and AST values. For the two protocols carried out, the animals that had received a treatment with collagen presented lower values of both markers (ALT and AST), which is very promising since it is known that a high protein intake (for example, casein) tends to increase transaminase levels.

**Table 8. Biochemical parameters of the experimental groups included in the study. Data are represented as group mean $\pm$ SEM. * p < 0.05 vs HFS; *** p < 0.001 vs HFS; ## p < 0.01 vs Casein 2h; b p < 0.05 vs col 24h; bb p < 0.01 vs col 24h. Note: Group 1 (Control) has not been considered in the statistical analysis, using Group 2 (HFS Control) as the reference group for 1-way ANOVAs.**

|  | CONTROL | HFS | CAS 24h | COL 24H | CAS 2H | COL 2H |
|---|---|---|---|---|---|---|
| Glucose (mg/dL) | 121.21 $\pm$ 1.28 | 129.07 $\pm$ 1.88 | 131.13 $\pm$ 2.31 | 132.18 $\pm$ 2.40 | 129.68 $\pm$ 2.28 | 126.56 $\pm$ 3.17 |
| Insuline ($\mu$g/L) | 0.75 $\pm$ 0.07 | 1.34 $\pm$ 0.18 | 1.47 $\pm$ 0.19 | 1.50 $\pm$ 0.28 | 1.47 $\pm$ 0.25 | 1.29 $\pm$ 0.28 |
| HOMA-IR | 5.59 $\pm$ 0.51 | 10.72 $\pm$ 1.57 | 11.76 $\pm$ 1.46 | 12.21 $\pm$ 2.34 | 11.87 $\pm$ 2.12 | 10.33 $\pm$ 2.60 |
| Cholestero l (mg/dL) | 71.20 $\pm$ 2.84 | 76.60 $\pm$ 3.66 | 76.50 $\pm$ 2.24 | 86.90 $\pm$ 2.89 | 71.70 $\pm$ 2.97 | 62.30 $\pm$ 2.81*,bb |
| HDL-COL (mg/dL) | 23.51 $\pm$ 0.88 | 23.34 $\pm$ 0.72 | 24.58 $\pm$ 0.56 | 25.72 $\pm$ 0.66 | 25.16 $\pm$ 0.88*** | 21.49 $\pm$ 0.74 ##, bb |
| Total Col/HDL-Col | 3.03 $\pm$ 0.06 | 3.27 $\pm$ 0.07 | 3.11 $\pm$ 0.06 | 3.38 $\pm$ 0.05 | 2.85 $\pm$ 0.05*** | 2.90 $\pm$ 0.07*** |
| AIP index | 0.45 $\pm$ 0.04 | 0.58 $\pm$ 0.04 | 0.52 $\pm$ 0.04 | 0.51 $\pm$ 0.03 | 0.39 $\pm$ 0.05* | 0.40 $\pm$ 0.05* |
| TG Serum (mg/dL) | 69.40 $\pm$ 8.19 | 91.10 $\pm$ 7.56 | 84.40 $\pm$ 8.30 | 86.20 $\pm$ 7.54 | 65.20 $\pm$ 8.23 | 57.00 $\pm$ 7.65* |
| TG Liver (mg/dL) | 4.46 $\pm$ 0.67 | 5.77 $\pm$ 0.78 | 6.09 $\pm$ 0.51 | 6.08 $\pm$ 0.46 | 4.82 $\pm$ 0.46 | 4.88 $\pm$ 0.52 |
| ALT (U/L) | 37.22 $\pm$ 1.81 | 43.35 $\pm$ 2.27 | 45.64 $\pm$ 2.25b | 36.79 $\pm$ 1.68 | 41.35 $\pm$ 1.98 | 37.04 $\pm$ 2.17 |
| AST (U/L) | 150.31 $\pm$ 8.480 | 183.34 $\pm$ 15.12 | 187.56 $\pm$ 12.43 | 148.43 $\pm$ 10.98 | 178.21 $\pm$ 11.65 | 164.64 $\pm$ 16.43 |

FINAL CONCLUSION:

At the macroscopic and biochemical level, no negative effects were observed that could be motivated by the intake of collagen. In fact, transaminase levels were lower in collagen supplemented animals than in casein supplemented animals. The results of the present study show that the collagen powder of the invention promotes an inhibition of the total daily intake of the animals, when it is supplemented in a single dose early in the morning. These satiating effects of collagen could be caused, at least in part, by its greater swelling capacity in the stomach, which would promote a lower post-prandial ghrelin release thus inhibiting food intake.

**Claims**

1. Dry collagen powder **characterized by**:

   a) more than 70% of the collagen is native;
   b) it has a gelatin content of less than 4% by weight;
   c) at least 95% of the powder has a granulometry of between 10 $\mu$m and 5 mm and it has a mean granulometry between 250 $\mu$m and 1 mm.

2. Powder according to claim 1 **characterized in that** it is made up of native collagen in more than 80%, more preferably in more than 90% and even more preferably in more than 95%.

3. Powder according to any of the preceding claims, **characterized in that** it has a gelatin content of less than 2.5% by weight, preferably less than 1% by weight, and even more preferably in the absence of gelatin.

4. Powder according to any of the preceding claims that has an undigested collagen fraction after the pepsin powder digestion test greater than 30%.

5. Powder according to any of the preceding claims, wherein the swelling percentage in the acid swelling test of collagen powders is greater than 1550%.

6. Powder according to any of the preceding claims wherein the collagen is of bovine, ovine, porcine, avian, fish origin or a mixture thereof.

7. Powder according to claim 6, wherein the collagen is of bovine origin and from an animal between 0 and 3 years old, preferably less than 2.5 years old.

8. Food or dietary supplement comprising collagen powder according to any of claims 1 to 7.

9. Food product comprising a powder according to any of claims 1 to 7 or a supplement according to claim 8.

10. Use of a powder according to any of claims 1-7, of a supplement according to claim 8 or of a food product according to claim 9 as a satiating agent in humans or animals.

11. Method for obtaining a collagen powder according to any of claims 1 to 7 comprising:

   a) mechanical treatment of a collagen source, previously conditioned and acidified, to reduce the size of the fibers until a mass of collagen is obtained;
   b) successive dilutions of the collagen mass of step a) at a temperature below 25 °C to obtain a concentrated collagen mass, with low or no gelation degree, maintaining the native state of the collagen;
   c) homogenization at a temperature below 25 ° C and extrusion of the collagen mass of step b);
   d) neutralization of the collagen mass of step c) with an alkaline agent to a pH between 6 to 9;
   e) drying the collagen mass of step d) to a moisture of between 1% and 18%; and
   f) grinding the dry mass of step d) until obtaining a collagen powder.

12. Method according to claim 11, wherein the conditioning and acidification treatment of the collagen source of step a) comprises:

   i. chemically removing the hair from the collagen source if the source used comprises them,
   ii. optionally, one or more washes with oxidizing agent,
   iii. optionally, alkaline treatment to remove fat and other impurities from the collagen source; and
   iv. acidification of the collagen source to a pH between 0.1 and 4.

**Patentansprüche**

1. Trockenes Kollagenpulver, **dadurch gekennzeichnet, dass**:

   a) mehr als 70 % des Kollagens nativ sind;
   b) es einen Gelatinegehalt von weniger als 4 Gew.-% aufweist;
   c) mindestens 95 % des Pulvers eine Granulometrie zwischen 10 $\mu$m und 5 mm aufweisen und es eine mittlere Granulometrie zwischen 250 $\mu$m und 1 mm hat.

2. Das Pulver gemäß Anspruch 1, **dadurch gekennzeichnet, dass** es zu mehr als 80 %, bevorzugt zu mehr als 90 % und noch bevorzugter zu mehr als 95 % aus nativem Kollagen ist.

3. Das Pulver gemäß einem der vorhergehenden Ansprüche, **dadurch gekennzeichnet, dass** es einen Gelatinegehalt von weniger als 2,5 Gew.-%, bevorzugt weniger als 1 Gew.-% und noch bevorzugter die Abwesenheit von Gelatine aufweist.

4. Das Pulver gemäß einem der vorhergehenden Ansprüche, das eine unverdaute Kollagenfraktion nach dem Pepsinpulververdauungstest von mehr als 30 % aufweist.

5. Das Pulver gemäß einem der vorhergehenden Ansprüche, wobei der Quellungsprozentsatz in dem Säurequellungstest von Kollagenpulvern mehr als 1550 % beträgt.

6. Das Pulver gemäß einem der vorhergehenden Ansprüche, wobei das Kollagen von einem Rinder-, Schaf-, Schwein-, Vogel-, Fischursprung oder einer Mischung davon ist.

7. Das Pulver gemäß Anspruch 6, wobei das Kollagen vom Rinderursprung ist und von einem Tier zwischen 0 und 3 Jahren, bevorzugt weniger als 2,5 Jahren ist.

8. Nahrungsmittel oder Nahrungsergänzungsmittel aufweisend Kollagenpulver gemäß einem der Ansprüche 1 bis 7.

9. Nahrungsmittelprodukt aufweisend ein Pulver gemäß einem der Ansprüche 1 bis 7 oder ein Nahrungsergänzungsmittel gemäß Anspruch 8.

10. Verwendung eines Pulvers gemäß einem der Ansprüche 1-7, eines Nahrungsergänzungsmittels gemäß Anspruch 8 oder eines Nahrungsmittelprodukts gemäß Anspruch 9 als Sättigungsmittel bei Menschen oder Tieren.

11. Ein Verfahren zum Erhalten eines Kollagenpulvers gemäß einem der Ansprüche 1 bis 7 aufweisend:

a) mechanische Behandlung einer Kollagenquelle, die zuvor konditioniert und gesäuert wurde, um die Größe der Fasern zu reduzieren, bis eine Kollagenmasse erhalten wird;
b) aufeinanderfolgende Verdünnungen der Kollagenmasse aus Schritt a) bei einer Temperatur unter 25 °C, um eine konzentrierte Kollagenmasse mit einem niedrigen oder keinem Gelierungsgrad zu erhalten, wobei der native Zustand des Kollagens beibehalten wird;
c) Homogenisierung bei einer Temperatur unter 25 °C und Extrusion der Kollagenmasse aus Schritt b);
d) Neutralisierung der Kollagenmasse aus Schritt c) mit einem alkalischen Mittel auf einen pH zwischen 6 und 9;
e) Trocknen der Kollagenmasse aus Schritt d) zu einer Feuchtigkeit zwischen 1 % und 18 %; und
f) Mahlen der trockenen Masse aus Schritt d), bis ein Kollagenpulver erhalten wird.

12. Das Verfahren gemäß Anspruch 11, wobei die Konditionierung und Säuerungsbehandlung der Kollagenquelle aus Schritt a) aufweist:

i. chemisches Entfernen der Haare von der Kollagenquelle, wenn die verwendete Quelle sie aufweist,
ii. optional eine oder mehrere Wäschen mit Oxidationsmittel,
iii. optional eine alkalische Behandlung, um Fett und andere Verunreinigungen von der Kollagenquelle zu entfernen; und
iv. Säuerung der Kollagenquelle zu einem pH zwischen 0,1 und 4.

**Revendications**

1. Poudre de collagène sèche **caractérisée par** :

a) plus de 70 % du collagène est natif ;
b) sa teneur en gélatine est inférieure à 4 % en poids ;
c) au moins 95 % de la poudre a une granulométrie comprise entre 10 $\mu$m et 5 mm et une granulométrie moyenne comprise entre 250 pm et 1 mm.

2. Poudre selon la revendication 1 **caractérisée en ce qu'**elle est composée de collagène natif à plus de 80%, plus préférablement à plus de 90% et encore plus préférablement à plus de 95%.

3. Poudre selon l'une quelconque des revendications précédentes, **caractérisée par** une teneur en gélatine inférieure à 2,5 % en poids, de préférence inférieure à 1 % en poids, et encore plus préférablement par l'absence de gélatine.

4. Poudre selon l'une quelconque des revendications précédentes dont la fraction de collagène non digérée après le test de digestion à la poudre de pepsine est supérieure à 30 %.

5. Poudre selon l'une quelconque des revendications précédentes, dans laquelle le pourcentage de gonflement dans le test de gonflement acide des poudres de collagène est supérieur à 1550%.

6.  Poudre selon l'une quelconque des revendications précédentes, dans laquelle le collagène est d'origine bovine, ovine, porcine, aviaire, piscicole ou un mélange de ceux-ci.

7.  Poudre selon la revendication 6, dans laquelle le collagène est d'origine bovine et provient d'un animal âgé entre 0 et 3 ans, de préférence de moins de 2,5 ans.

8.  Complément alimentaire ou diététique comprenant de la poudre de collagène selon l'une quelconque des revendications 1 à 7.

9.  Produit alimentaire comprenant une poudre selon l'une quelconque des revendications 1 à 7 ou un supplément selon la revendication 8.

10. Utilisation d'une poudre selon l'une quelconque des revendications 1 à 7, d'un supplément selon la revendication 8 ou d'un produit alimentaire selon la revendication 9 comme agent rassasiant chez l'homme ou l'animal.

11. Procédé d'obtention d'une poudre de collagène selon l'une quelconque des revendications 1 à 7, comprenant :

     a) traitement mécanique d'une source de collagène, préalablement conditionnée et acidifiée, pour réduire la taille des fibres jusqu'à l'obtention d'une masse de collagène ;
     b) dilutions successives de la masse de collagène de l'étape a) à une température inférieure à 25 °C pour obtenir une masse de collagène concentrée, avec un degré de gélification faible ou nul, en conservant l'état natif du collagène ;
     c) homogénéisation à une température inférieure à 25°C et extrusion de la masse de collagène de l'étape b) ;
     d) neutralisation de la masse de collagène de l'étape c) à l'aide d'un agent alcalin jusqu'à un pH compris entre 6 et 9 ;
     e) séchage de la masse de collagène de l'étape d) jusqu'à une humidité comprise entre 1 % et 18 % ; et
     f) broyage de la masse sèche de l'étape d) jusqu'à l'obtention d'une poudre de collagène.

12. Procédé selon la revendication 11, dans lequel le traitement de conditionnement et d'acidification de la source de collagène de l'étape a) comprend :

     i. le retrait chimique des poils de la source de collagène si la source utilisée en comporte,
     ii. optionnellement, un ou plusieurs lavages avec un agent oxydant,
     iii. optionnellement, un traitement alcalin pour éliminer la graisse et les autres impuretés de la source de collagène ; et
     iv. acidification de la source de collagène à un pH compris entre 0,1 et 4.

**Fig. 1A**

## Fig. 1B

**Fig. 1C**

Fig. 1D

# Fig. 2

A

B

C

D

**Fig. 3**

**Fig. 4**

**Fig. 5**

**Fig. 6**

**Fig. 7**

FIG. 8

**Fig. 9**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 10161986
- WO 2009049105 A
- WO 2004056375 A
- WO 2004056393 A
- EP 2651426 A
- KR 100417020
- EP 0901792 A
- CN 106509126
- EP 2575496 A
- CN 107912543 A

### Non-patent literature cited in the description

- Soluble collagen and the components resulting from its denaturation. **PIEZ**. Treatise on Collagen. Academic Press, 1967, vol. 1, 207